# EUROPÄISCHE PATENTSCHRIFT

(11) **EP 1 972 602 B1**
(45) Veröffentlichungstag und Bekanntmachung des Hinweises auf die Patenterteilung: **14.03.2012**
(21) Anmeldenummer: 08003541.3
(22) Anmeldetag: 27.02.2008
(51) Int. Cl.: A01G 33/00, A01H 4/00, C12M 1/00

(54) **Verfahren zur Erzeugung von Biomasse unter Verwendung photosynthetischer Algenmischkulturen**
Method of generating biomass by making use of photosynthetically active mixed algae culture
Procédé de production de biomasse en utilisant des cultures mixtes d'algues photosynthetiques

(30) Priorität: 15.03.2007 DE 102007012409
(43) Veröffentlichungstag der Anmeldung: 24.09.2008
(73) Patentinhaber: Holm, Niels Christian, 32425 Minden (DE)
(72) Erfinder: Holm, Niels Christian, 32425 Minden (DE)
(74) Vertreter: Tönnies, Jan G.

(56) Entgegenhaltungen:
- DE-C1- 3 812 781
- FR-A- 2 874 006
- US-A- 4 267 038
- US-A- 5 338 673
- US-A1- 2002 034 817
- 19000101, 1. Januar 1900 (1900-01-01), XP009101993

## Beschreibung

Die Erfindung betrifft ein Verfahren zur Biomasseproduktion unter Verwendung photosynthetischer Algenmischkulturen.

Biomasse wird nach unterschiedlichster Aufbereitung zu Nahrungsmittelzwecken verwendet, aus ihr werden aber auch die unterschiedlichsten Produkte gewonnen wie Feinchemikalien, Vitamine, Farbstoffe etc.

Im Rahmen der Abwasserreinigung wird die anfallende Biomasse als zu entsorgender Feststoff behandelt, der z.B. über die Faulung entsorgt werden kann oder im Rahmen von Biogasanlagen als nachwachsender Rohstoff zur Biogasproduktion eingesetzt werden kann.

Unabhängig vom Verwendungszweck muss bei den bisherigen Kulturtechnologien die Algenbiomasse immer aus den wässrigen Kulturmedien aufkonzentriert entnommen werden.

Es ist bekannt, dass Algenmassen- bzw. Algenmischkulturen unterschiedlichster Anlagenkonfigurationen zur Biomasseproduktion und/oder Abwasserreinigung in Kombination mit Biogasanlagen (z.B. DE 197 21 243 C2) eingesetzt werden.

Die DE 38 12 781 C1 beschreibt die Nachklärung von Abwasser in einem eine Algenmischkultur enthaltenden Rundbecken. Die US-A-5 338 673 lehrt das Kultivieren von Mikroalgen, wobei die Ernte in einem Trichter erfolgt. Die FR-A-2 874 006 beschreibt die Kultivierung von planktonischen Algen/Bakterienkulturen in einem geschlossenen Rundbecken, wobei die Entnahme n einem unteren konischen Bereich erfolgt.

Aus den unterschiedlichsten Gründen sind die meisten der bisherigen Groß-Kulturtechniken darauf ausgerichtet gewesen, planktische Algen zu kultivieren, die sich nicht absetzen, und daher recht aufwendig aus der wässrigen Lösung mittels Filtration, Zentrifugation etc. aufkonzentriert werden müssen. Mit Ausnahme sehr teurer tubulärer, geschlossener Systeme (die z.B. für die Reinkulturzüchtung eingesetzt werden) leiden alle bisherigen offenen Systeme darunter, dass mehr oder weniger große Biomassefraktionen sich doch absetzen und die Produktion der rein planktischen Fraktionen mehr oder weniger stark hemmen.

Andere Großkulturtechniken (z.B. zur Abwasserreinigung) sind darauf ausgerichtet möglichst nur eine sich absetzende Algenbiomasse zu kultivieren. Auf Grund ungünstiger hydraulischer Rahmenbedingungen gelingt dies jedoch nur unzureichend; des Weiteren sind Aufwendungen zur in Schwebe-Haltung dieser Biomasse und Gewährleistung ausreichender Kontaktzeiten mit dem Nährmedium sehr viel aufwendiger als für planktische Kulturen.

Abgesehen von einer Reihe weiterer technologischer Probleme (wie z.B. ausreichende CO₂ Zufuhr bzw. Vermeidung von zu starken O₂ Übersättigungen) sind die beiden genannten Aspekte, nämlich zum einen unzureichende Erntetechniken und zum anderen unzureichende Selektionstechniken für planktische oder sedimentierbare Algenbiomasse die Hauptursache für den bis zum heutigen Tage praktisch nicht vorhandenen großtechnischen Einsatz dieser Technologie.

Dies ist umso bemerkungswerter, als das mit dieser Technologie die Produktivität in kg Trockensubstanz/(m² Jahr) um ein mehrfaches höher liegt als mit allen heute angebauten Kultur- und Wildpflanzen, inklusive Raps, Hochleistungsmaissorten etc. für die Biogas- oder Biokraftstoffproduktion, die auf Basis der deutschen Energie-Einspeise-Gesetzgebung (EEG) gefördert wird.

Der Erfindung liegt damit die Aufgabe zugrunde, ein Verfahren zur Durchführung einer Algenmasseproduktion zu schaffen, bei der die Aufwendungen für die Biomasse - Ernte gering sind oder völlig entfallen und eine zielgerichtete Produktion nur planktischer oder nur absetzbarer Algenmischkulturen gewährleistet ist.

Erfindungsgemäß wird diese Aufgabe wird in Bezug aus planktischen Algen-/Bakterienmischkulturen durch die Merkmale des Anspruchs 1 und in Bezug auf sedimentierbaren Algen-/Bakteriennlischkulturen durch die Merkmale des Anspruchs 5 gelöst, die Unteransprüche geben vorteilhafte Ausgestaltungen der Erfindung an.

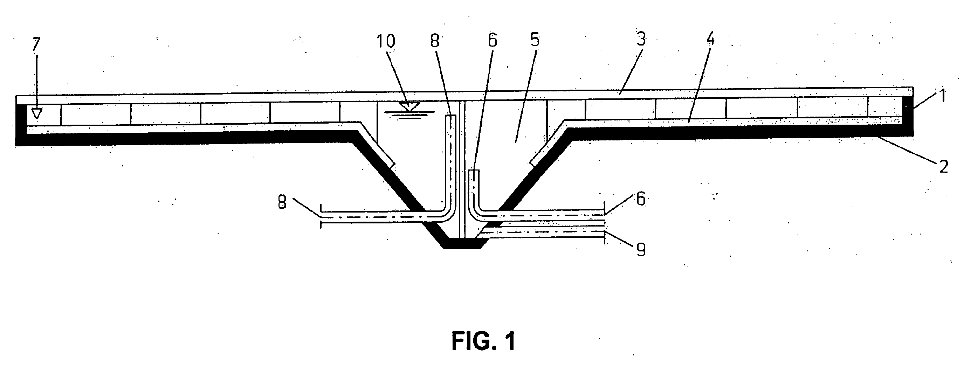

Die Erfindung wird im Folgenden anhand einer Zeichnung erläutert. Dabei zeigt Fig. 1 einen Schnitt durch eine entsprechende Einrichtung zur Kultivierung planktischer Algen. (eine Einrichtung zur Kultivierung sedimentierbarer Algen wird analog ausgeführt).

Zur Kultivierung von ausschließlich planktischen Algenpopulationen (bei denen ein bedeutender Anteil auch aus Bakterien bestet) sind offene, flache Rundbecken 1 vorgesehen mit horizontalen Sohlen 2 und Räumeinrichtungen 3 mit Schildräumern 4, die den Schildräumer für runde Nachklärbecken beim Belebtschlammverfahren in der Abwasserreinigung entsprechen, die kontinuierlich die sedimentierten Anteile (Algen oder andere partikuläre Bestandteile) wieder in Schwebe bringen. Auf Basis einer solchen Ausführungsform mit den schnellsten Räumgeschwindigkeiten an der Peripherie werden sedimentierbare Anteile umso weniger in Schwebe gehalten, je näher sie am Zentrum sind.

Das Zentrum eines solchen Beckens ist als Trichter 5 ausgebildet, aus dem an drei unterschiedlichen Stellen Entnahmen erfolgen können:
1) Aus dem freien Wasser 5 bis 60 cm unter dem Wasserspiegel und 30 bis 200 cm über der Trichterspitze wird über ein Rohr 6 überwiegend kontinuierlich (zumindest während der photosynthetisch aktiven Zeiten) die planktische Algensuspension entnommen und tangential an den Beckenrand zurückgefördert. In Kombination mit der Räumung und der peripheren Zufuhr an Nährlösung 7, z.B. ausgegorenes Substrat aus einem Biogasfermenter, erfolgt damit ein starker positiver Selektionsdruck auf die planktischen Algenfraktionen. Ein Teil der Entnahmen kann auch über diese Stelle erfolgen, überwiegend müssen die Entnahmen allerdings über ein Rohr 9, das an der Spitze des Trichters 5 liegt, erfolgen.
   Bei dieser Betriebsweise werden zudem alle flottierenden und sedimentierbaren Algenbiomassefraktionen von der Peripherie weg in Richtung Zentrum verschoben.
2) Aus einem zentral angebrachtem Überlaufrohr 8 in Höhe des Wasserspiegels 10 wird periodisch eine geringe Fraktion entnommen und extern entsorgt. Damit werden flottierende Algenbiomassefraktionen (z.B. Arten mit Gasvesikeln wie einige Cyanobakterien) einem starken negativen Selektionsdruck ausgesetzt und somit praktisch quantitativ aus dem System entfernt.
3) Aus der Spitze des Trichters 5 erfolgen über das Rohr 9 die überwiegenden Entnahmen aus dem System zur externen Weiterverarbeitung. Damit erfolgt ein stark negativer Selektionsdruck auf die sedimentierbaren Algenbiomassefraktionen, die somit praktisch quantitativ eliminiert werden.

Als Ergebnis der obigen Betriebsweise stellt sich eine rein planktische Algenpopulation ein ohne nennenswerte sedimentierbare oder flottierbare Anteile. Es ist Stand der Wissenschaft, dass eine solche planktische Mischkultur die höchsten photosynthetischen Erträge überhaupt erzielt. Für die Verwendung einer solchen planktischen Kultur zur Verfütterung an eine Zooplanktonkultur ist überhaupt keine Ernte erforderlich. Auch für die Verwertung als nachwachsender Rohstoff (in Ergänzung zu beispielsweise Silagemais) in Biogasanlagen ist in vielen Fällen keine Ernte erforderlich.

Im Rahmen einer besonderen Ausführungsform der Erfindung werden ausgegorene und abgesetzte Gärreste (oder auch Faulschlamm aus Anaerob-Behandlungsanlagen) einem solchen Algenbecken peripher, tangential zugeführt (dabei kann optional eine Vorbehandlung in Form einer Faulschlamm-/Gärrestwaschung erfolgen oder auch nicht). Diese Gärreste/Faulschlämme bestehen überwiegend aus strikt anaeroben Bakterien (z.B. den extrem sauerstoffempfindlichen Methanbakterien). Nach Zufuhr in ein solches Algenbecken werden diese Anaerobbakterien hohen SauerstoffKonzentrationen ausgesetzt und zu einem erheblichen Anteil abgetötet. Damit werden sie in Substrat für eine Biogasanlage umgewandelt. Ein Teil dieser getöteten Bakterien wird lysieren und den Zytoplasma-Inhalt an das Medium abgeben. Dieses dient als Substrat, sowohl bezüglich Kohlenstoff/CO2 als auch aller erforderlichen Nährsalze, für die planktische Algen-/Bakterienmischkultur. Der Rest der zugefiihrten Biomasse-Substrate wird letztendlich im zentralen Trichter sedimentieren und daher konzentriert daraus entnommen zur "Wiederverwendung" in der AnaerobBehandlung.

Mit dieser Betriebsweise wird gewährleistet, dass gleichzeitig nur eine verhältnismä-ßig geringe Menge an der ursprünglichen planktischen Algenpopulation entnommen wird. Hinsichtlich der Biomassemengen kann somit eine viel größere Menge an Gärreststoffen durch die Anlage gefahren und anschließend aus dem zentralen Trichter entnommen werden, als im gesamten Becken an Algenbiomasse vorhanden ist. Die Betriebsweise ist damit darauf ausgelegt, maximal so viel Gärreste pro Tag durch das Algenbecken zu fahren, wie mit der anschließenden Entnahme auch an Netto-Algenbiomassezuwachs (Überschuss) entnommen wird.

Im Allgemeinen wird der Algenbiomassezuwachs deutlich höher sein.

Gesteuert / geregelt werden die Gärrestdurchsatzmengen über die Sauerstoffkonzentration im Algenbecken: Nach Unterschreitung eines gewählten O₂-Sollwertes wird die Gärrestzufuhr unterbrochen. Damit wird gewährleistet, dass die maximal möglichen Menge an Gärresten verarbeitet werden, ohne dass der Algenanteil der planktischen Algen-/Bakterienmischpopulation zu stark verringert wird, und daher nachfolgend nicht mehr die maximal mögliche Sauerstoffmenge produziert werden kann. Der verbleibende Netto-Überschuss kann (siehe oben) ohne Einsatz von Erntetechniken direkt an Zooplankton verfüttert werden.

Im Rahmen einer weiteren besonderen Ausführungsfonn der Erfindung kann dieser Netto-Überschuss als Bestandteil eines Gärrrestrecyclings in diesen unter Verdrängung einer volumengleichen Trübwassermenge aus den Gärresten hineingewaschen werden, um die Zufuhr in die Biogas-/Faulungsanlage trotz Verwendung einer vergleichsweise sehr "dünnen" Algensuspension mit üblicherweise weniger als 0,1 - 3 g TS/1 nicht zu verdünnen.

Im Ergebnis erfolgt eine Produktion an nachwachsenden Rohstoffen auf Basis einer planktischen Algen-/Bakterienmassenkultur sowie gleichzeitig die Umwandlung einer viel größeren Menge an ausgegorenen (d.h. ohne nennenswertes weiteres Biogaspotential) Gärresten zurück in eine Form, die wieder als Ausgangssubstrat (d.h. mit einem viel höheren Biogaspotential) für die Biogasanlage dienen kann.

Unmittelbar erkennbar ist, dass im Extremfall das gesamt organische Ausgangsmaterial in Biogas umgewandelt werden kann und nur noch konzentriertes Trübwasser aus der Anaerobbehandlung anfällt.

### Beispiel:

In einem Algenbecken wie oben beschrieben mit 10.000 m² Nutzfläche, einem Wasserspiegel von 6 cm (= 600 m³ Algensuspension) und einer planktischen Algen/Bakteriendichte von 100 g oTS/m² (= 1,67 g/1= 1.000 kg oTS)) ergibt sich unter günstigen klimatischen Vorraussetzungen sowie einer optimalen Nährstoffversorgung über die Gärrestezufuhr eine Nettotagesproduktion von 40 g oTS/m² = 400 kg oTS/Tag. Der O₂ Sollwert ist so hoch eingestellt, dass sich eine Nettosauerstoffproduktion ergibt, die zu einer gewissen O₂-Übersättigung des Mediums führt.

Dem Becken werden pro Tag 100 m³ Gärreste mit 4 % oTS (= 4.000 kg oTS) zugeführt. Der Einfachheit halber wird davon ausgegangen, dass hiervon 400 kg oTS pro Tag lysieren und die Grundlage für die Produktion der 400 kg oTS/Tag planktischen Algen-/Bakterienbiomasse sind.

Nach einem eintägigen Produktionszyklus ergibt die Bilanz 3.600 kg oTS Gärrest und 400 kg/d oTS zusätzlich Algenbiomasse, die entnommen werden müssen. Der teilweise in Substrat umgearbeitete Gärrest wird aus der Trichterspitze mit 3,6 % TS = 100 m³ entnommen. Damit ist auch 1/7 der gesamten planktischen Algenbiomesse entnommen worden = 200 kg oTS. Die verbleibenden 200 kg oTS müssen auch noch entfernt werden. Da in den verbleibenden 600 m³ noch 1.200 kg oTS vorhanden sind (= 2 g/l), müssen genau noch 100 m³ reine planktische Algenbiomasse entnommen werden (die rein hydraulisch durch externe Zufuhr von z.B. 100 m³ Trübwasser aus der Faulung ersetzt werden müssen), beispielsweise durch Hineinwaschen in einen zusätzlichen Gärrestrecyclingstrom. Die Regelung dieser Entnahme erfolgt sinnvollerweise mittels einer online TS-Messung in Verbindung mit einer Höhenstandsmessung.

Das Biogaspotential der planktischen Algenbiomasse kann mit ca. 1.000 1 Biogas (ca. 70 % Methan) pro kg zugeführtem oTS abgeschätzt werden. Das wären pro Tag 400 m³ Biogas, d.h. 400 m³/(Hektar · Tag).

Während das Biogaspotential des ausgegasten Gärrestes bei nur noch ca. 20 l Biogas (ca. 70 % Methan) pro kg zugeführtem oTS liegt, steigt es nach Durchlauf durch das Algenbecken (im Wesentlichen auf Grund der Teilabtötung der anaeroben Bakterien) auf abgeschätzte 200 l Biogas (ca. 70 % Methan) pro kg zugeführtem oTS. Dieses ist nur noch zu beziehen auf die entnommenen 3.600 kg oTS/Tag. Das ergibt somit zusätzliche 720 m³ Biogas/(Hektar . Tag).

In Summe ergeben sich ca. 1.120 m³ Biogas/(Hektar · Tag). Das ist ca. das 40 fache dessen, was unter temperierten klimatischen Bedingungen im Jahresmittel mittels Maisanbau pro Hektar und Verwertung in einer Biogasanlage an Biogas produziert werden kann. Bezogen aufs Jahresmittel unter temperierten Klimabedingungen wird die oben beschriebene Algenkulturtechnik allerdings nur ca. den halben Ertrag erbringen. Das ist aber immer noch das 20 fache des Maisertrages.

Zur Kultivierung von ausschließlich sedimentierbaren Algenpopulationen (bei denen ein bedeutender Anteil auch aus Bakterien bestehen kann) sind wiederum offene, flach Rundbecken vorgesehen mit horizontalen Sohlen und Räumeinrichtungen (analog Schildräumer für runde Nachklärbecken beim Belebtschlammverfahren in der Abwasserreinigung), die kontinuierlich die sedimentierten Anteile (seien dies Algen oder andere partikuläre Bestandteile) wieder in Schwebe bringen. Auf Basis einer solchen Ausführungsform mit den schnellsten Räumgeschwindigkeiten an der Peripherie werden sedimentierbare Anteile umso weniger in Schwebe gehalten, je näher sie am Zentrum sind.

Das Zentrum eines solchen Beckens wird wiederum als Trichter ausgebildet, aus dem an drei unterschiedlichen Stellen Entnahmen erfolgen können:
1) Aus einem zentral angebrachtem Überlaufrohr in Wasserspiegelhöhe wird periodisch eine geringe Fraktion entnommen und extern entsorgt. Damit werden flottierende Algenbiomassefraktionen (z.B. Arten mit Gasvesikeln wie einige Cyanobakterien) einem starken negativen Selektionsdruck ausgesetzt und somit praktisch quantitativ aus dem System entfernt. Daher dient diese Entnahmestelle auch der Entnahme der nahezu feststoffreien Klarwasserfraktion, die je nach den Rahmenbedingungen des Systems Vorfluterqualität besitzt und direkt in eine Vorfluter abgeleitet werden kann oder weiterverarbeitet werden kann oder muss.
2) Aus der Trichterspitze wird überwiegend kontinuierlich (zumindestens während der photosynthetisch aktiven Zeiten) die sedimentierte Algensuspension entnommen und tangential an den Beckenrand zurückgefördert. In Kombination mit der Räumung und der peripheren Zufuhr an Nährlösung (z.B. überwiegend feststoffreies Trübwasser aus einem Biogasfermenter) erfolgt damit ein starker positiver Selektionsdruck auf die sedimentierbaren Algenfraktionen. Aus dieser Entnahmestelle wird die ganze produzierte Algenbiomasse entnommen.
   Mit dieser Form der Betriebsweise werden zudem alle flottierenden und sedimentierbaren Algenbiomassefraktionen von der Peripherie weg in Richtung Zentrum verschoben.
   Falls die hydraulische Aufenthaltszeit in System nicht deutlich geringer ist als die Aufenthaltszeit der sedimentierbaren Algenfraktionen, werden sich trotz dieser Betriebsweise auch planktische Algenfraktionen herausbilden, ansonsten nicht. In solchen Fällen muss die hydraulische Aufenthaltszeit künstlich reduziert werden, so dass die reaktive hydraulische Aufenthaltszeit so viel geringer ist als die der sedimentierbaren Fraktionen, dass planktische Fraktionen sich nicht etablieren können. Dazu dient die dritte Entnahmestelle.
3) Aus dem freien Wasser 5 bis 30 cm unter dem Wasserspiegel und 30 bis 200 cm über der Trichterspitze wird periodisch oder kontinuierlich (zumindestens während der photosynthetisch aktiven Zeiten) eine so hohe Menge entnommen und entweder
   A: unter Lichtabschluss so lange zwischengespeichert (und mit Beginn der Dunkelperiode zurückgefördert), dass die resultierende hydraulische Aufenthaltszeit so gering wird, dass sich keine plastischen Algenpopulationen etablieren können,
      oder
   B: über eine Filtration von planktischen Anteilen befreit und das Klarwasser zurück ins Becken gefördert, so dass sich planktische Fraktionen nicht in nennenswerten Mengen etablieren können.

Alternativ können auch aus der Entnahmestelle 1) diese beiden Prozesse durchgeführt werden.

Als Ergebnis der obigen Betriebsweisen stellt sich eine rein sedimentierbare Algenpopulation ein ohne nennenswerte planktische oder flottierbare Anteile. Für diese sedimentierte Algenbiomasse sind meistens keinerlei weitere Ernte-/Anreicherungsmaßnahmen erforderlich, da durch die Eindickung ein ausreichend hoher TS-Gehalt erreicht werden kann. Ansonsten verbleibt nur eine Klarwasserfraktion, die für die unterschiedlichsten Verwendungszwecke wie Verregnung, Ableitung in die Vorflut etc. genutzt werden kann.

Im Rahmen einer besonderen Ausführungsform der Erfindung wird bei der sedimentier-Betriebsweise eine Filtration der planktischen Klarwasserphase durchgeführt, indem diese Fraktion durch die Gärreste einer Faulung "hindurchgewaschen" werden, wobei das verdrängte Trübwasser ins Algenbecken geführt wird und die Algen in der Gärrestfraktion verbleiben.

Im Rahmen dieser besonderen Ausführungsform der Erfindung wird bei der sedimentier-Betriebsweise die Klarwasserphase dazu genutzt, durch einen Gärrestrecyclingstrom "hindurchgewaschen" zu werden: Die hohe O₂ Konzentration im Klarwasser führt zu einem Teilaufschluss dieser Gärreste und somit zu einer Erhöhung ihres Gasbildungspotentials. Nach dieser Waschung werden diese Gärreste zurück in die Faulung gefördert.

Selbst die sedimentierte Algenfraktion wird eine vergleichsweise geringe TS-Konzentration von ca. 1,5 - 3 % TS aufweisen. Das ist als Ausgangssubstrat für eine Biogasanlage hydraulisch ungünstig.

Im Rahmen einer weiteren besonderen Ausführungsform der Erfindung wird bei der Sedimentier-Betriebsweise die entnommene sedimentierte Algenfraktion von Trübwasser aus dem Ablauf der Biogasanlage durchspült, und somit eine gleiche Klarwassermenge verdrängt (die üblicherweise ins Algenbecken zurückgeführt wird). Das bewirkt, dass keinerlei Netto-Zufuhr (und keinerlei Verdünnung) in die Biogasanlage erfolgt ist, sondern nur eine Zufuhr der reinen Algenbiomasse.

Die wässrigen Zufuhrfraktionen zur Faulungsanlage können mit sedimentierbaren Bestandteilen (insbesondere Belebtschlamm aus Abwasserreinigungsanlagen) von Trübwassern aus dem Ablauf einer Biogasanlage durchspült, bevor sie in die Faulungsanlage gefördert werden. Damit wird eine volumengleiche Wassermenge verdrängt. Diese wird üblicherweise ins Algenbecken oder in die Abwasserreinigungsanlage zurück geführt.

## Patentansprüche

1. Verfahren zur Massenkultivierung von ausschließlich planktischen Algen-/Bakterienmischkulturen in offenen, flachen, mit Schildräumeinrichtungen (3) versehenen Rundbecken unter peripherer Zufuhr von Nährlösungen (7) und einem zentralen Entnahmetrichter mit drei Entnahmestellen, bei dem zur Kultivierung von Algen-/Bakterienmischkulturen über einen zentralen Überlauf (8) periodisch flottierende Bestandteile entnommen werden, über eine untere Entnahmestelle (9) auf Höhe der Trichtersohle sich absetzende Fraktionen entnommen werden und über eine zwischen dem Überlauf und der unteren Entnahmestelle (9) liegende mittlere Entnahmestelle (6) die planktische Algen-/Bakterienfraktion aus dem freien Wasser entnommen und an die Peripherie des Beckens zurückfördert wird.

2. Verfahren nach Anspruch 1, **dadurch gekennzeichnet, dass** ausgegorene Gärreste oder Faulschlämme, überwiegend bestehend aus strikt anaeroben Bakterien, dem Algenbecken peripher zugeführt werden und nachdem diese Biomassefraktion in den zentraler Trichter gelangt ist, von dort entnommen und in die Anaerob-Behandlungsanlage zurückgeführt wird.

3. Verfahren nach Anspruch 2 oder 3, **dadurch gekennzeichnet, dass** die Menge an zugeführten Gärresten O₂ geregelt erfolgt.

4. Verfahren nach einem der. Anspruch 1 bis 3, **dadurch gekennzeichnet, dass** die planktische Überschuss-Menge zur Vorbereitung für eine Verwertung in einer Anaerob-Behandlungsanlage in eine ausgegorene Gärrestfraktion hineingewaschen wird.

5. Verfahren zur Massenkultivierung von ausschließlich sedimentierbaren Algen-/Bakterienmischkulturen in offenen, flachen, mit Schildräumeinrichtungen (3) versehenen Rundbecken unter peripherer Zufuhr (7) von Nährlösungen und einem zentralen Entnahmetrichter mit drei Entnahmestellen, bei dem über einen zentralen Überlauf (8) periodisch flottierende Bestandteile und Klarwasser entnommen werden, über eine untere Entnahmestelle (9) auf Höhe der Trichtersohle sich absetzende Fraktionen entnommen werden und - ohne die produzierte Überschussbiomasse - an die Peripherie des Beckens zurückfördert wird und über eine zwischen dem Überlauf und der unteren Entnahmestelle (9) liegende mittlere Entnahmestelle (6) die planktische Algen-/Bakterienfraktion in so großen Mengen aus dem freien Wasser entnommen wird, dass diese sich nicht etablieren kann.

6. Verfahren nach Anspruch 5, **dadurch gekennzeichnet, dass** die Klarwasserfraktion durch die Gärreste einer Faulung "hindurchgewaschen" werden und die **dadurch** teilaufgeschlossenen Gärreste zurück in die Faulung gefördert werden.

7. Verfahren nach Anspruch 5 oder 6, **dadurch gekennzeichnet, dass** die sedimentierte Algenbiomasse mit der Trübwasserfraktion der Gärreste durchspült wird, bevor sie in die Faulung gefördert wird.

8. Verfahren nach einem der vorangehenden Ansprüche, **gekennzeichnet durch** Durchspülen einer wässrigen Zufuhrfraktionen in einer Faulungsanlage mit sedimentierbaren Bestandteilen, insbesondere Belebtschlamm aus Abwasserreinigungsanlagen, von Trübwasser aus dem Ablauf der Faulungsanlage, bevor sie in die Faulungsanlage gefördert werden.

## Claims

1. A method for mass cultivation of exclusively planktic mixed algae/bacteria cultures in open, flat round basins provided with shield scrapers (3) while peripherally feeding culture media (7) and a central extraction funnel with three extraction points, wherein for cultivating mixed algae/bacteria cultures floating components are periodically extracted via a central overflow (8), fractions settling at the level of the funnel floor are extracted via a lower extraction point (9), and the planktic algae/bacteria fraction is extracted from the free water and fed back to the periphery of the basin via a central extraction point (6) disposed between the overflow and the lower extraction point (9).

2. The method according to Claim 1, **characterized in that** the fermented fermentation residues or digested sludge, predominantly consisting of strictly anaerobic bacteria, are peripherally fed to the algae basin and are removed from there, after this biomass fraction has arrived in the central funnel, and fed back into the anaerobic treatment plant.

3. The method according to Claim 2 or 3, **characterized in that** the amount of fermentation residues is fed controlled in terms of O₂.

4. The method according to one of Claim 1 to 3, **characterized in that** for preparation for utilisation in an anaerobic treatment plant, the planktic excess amount is rinsed into a fermentation-residue fraction that has finished fermenting.

5. The method for mass cultivation of exclusively sedimentable mixed algae/bacteria cultures in open, flat round basins provided with shield scrapers (3) while peripherally feeding (7) culture media and a central extraction funnel with three extraction points, wherein floating components and clear water are periodically extracted via a central overflow (8), fractions settling at the level of the funnel floor are extracted via a lower extraction point (9) and fed back - without the excess biomass produced - to the periphery of the basin, and the planktic algae/bacteria fraction is extracted via a central extraction point (6) disposed between the overflow and the lower extraction point (9) in such amounts from the free water that it cannot establish itself.

6. The method according to Claim 5, **characterized in that** the clear-water fraction is "rinsed through" the fermentation residues of a fermentation and the fermentation residues that are partially solubilised thereby are transported back into the fermentation stage.

7. The method according to Claim 5 or 6, **characterized in that** the sedimented algae biomass is flushed through with the turbid-water fraction of the fermentation residues before it is transported into the fermentation stage.

8. The method according to one of the preceding claims, **characterized by** flushing an aqueous feeding fraction in a fermentation plant with sedimentable components, in particular activated sludge from wastewater treatment plants, from turbid water from the runoff of the fermentation plant before being transported into the fermentation plant.

## Revendications

1. Procédé pour la cultivation de masse de cultures mélangées d'algues/de bactéries, exclusivement planctiques dans des bassins circulaires, ouverts et plats, pourvus d'installations de nettoyage par bouteur (3), avec une alimentation phériphérique de solutions nutritives (7) et une trémie d'extraction centrale avec trois points de prélèvement, pour lequel pour la cultivation de cultures mélangées d'algues/de bactéries, on prélève périodiquement, via un débordement central (8), des composants flottants, on prélève, via un point inférieur de prélèvement (9) des fractions, qui se déposent à hauteur du fond de trémie, et via un point central de prélèvement (6), se situant entre le débordement et le point inférieur de prélèvement (9), la fraction d'algues/de bactéries planctique de l'eau libre et ramenée à la phériphérie du bassin.

2. Procédé d'après revendication 1, **caractérisé en ce que** des restes de fermentation ou sapropels fermentés, de façon prépondérante consistant en des bactéries strictement anaérobiques sont amenées vers le bassin d'algues de manière périphérique et une fois que cette fraction de biomasse est parvenue à la trémie centrale, y est prélevée et ramenée dans l'installation de traitement anaérobique.

3. Procédé d'après revendication 2 ou 3, **caractérisé en ce que** la quantité des restes de fermentation rajoutée s'effectue de façon réglée par O₂.

4. Procédé d'après l'une des revendications 1 à 3, **caractérisé en ce que** la quantité en excès planctique, pour une préparation pour une utilisation dans une installation de traitement anaérobique, est lavée vers une fraction de restes de fermentation.

5. Procédé pour la cultivation de masse de cultures mélangées d'algues/de bactéries, exclusivement sédimentables es dans des bassins circulaires, ouverts et plats, pourvus d'installations de nettoyage par bouteur (3), avec une alimentation phériphérique de solutions nutritives (7) et une trémie d'extraction centrale avec trois points de prélèvement, pour lequel pour la cultivation de cultures mélangées d'algues/de bactéries, on prélève périodiquement, via un débordement central (8), des composants flottants et de l'eau décantée, on prélève, via un point inférieur de prélèvement (9) des fractions, qui se déposent à hauteur du fond de trémie et on les ramène à la périphérie du bassin--sans la biomasse en excès produite, et via un point central de prélèvement (6), se situant entre le débordement et le point inférieur de prélèvement (9), on prélève la fraction d'algues/de bactéries planctique de l'eau libre dans des quantités suffisamment grandes, afin que celle-ci ne puisse pas s'établir.

6. Procédé d'après revendication 5, **caractérisé en ce que** la fraction d'eau décantée est "lavée à travers" les restes de fermentation d'une macération et que de ce fait les restes de fermentation partiellement ouvertes sont retournées dans la macération.

7. Procédé d'après revendication 5 ou 6, **caractérisé en ce que** la biomasse d'algues sédimentée est rincée avec la fraction d'eaux troubles des restes de fermentation, avant d'être retournée dans la macération.

8. Procédé d'après l'une des revendications précédentes, **caractérisé par** le rinçage d'une fraction amenée aqueuse dans une installation de macération avec des composants sédimentables, en particulier de la boue activée d'installations d'épuration des eaux usées, d'eaux troubles de l'écoulement de l'installation de macération, avant d'être d'y être transportée.
